# EUROPÄISCHE PATENTSCHRIFT

(11) **EP 3 285 698 B1**
(45) Veröffentlichungstag und Bekanntmachung des Hinweises auf die Patenterteilung: **01.11.2023**
(21) Anmeldenummer: 16726484.5
(22) Anmeldetag: 20.04.2016
(51) Int. Cl.: A61F 2/64, A61F 5/01

(54) **FÜHRUNGSGELENK FÜR EINE GELENKSORTHESE**
HINGE FOR AN ARTICULATION ORTHOSIS
JOINT POUR UNE ORTHÈSE D'ARTICULATION

(30) Priorität: 22.04.2015 AT 503232015
(43) Veröffentlichungstag der Anmeldung: 28.02.2018
(73) Patentinhaber: Wayd, Kurt, 1020 Wien (AT)
(72) Erfinder: Wayd, Kurt, 1020 Wien (AT)
(74) Vertreter: Patentanwälte Pinter & Weiss OG
(86) Internationale Anmeldenummer: PCT/EP2016/058716
(87) Internationale Veröffentlichungsnummer: WO 2016/169960

(56) Entgegenhaltungen:
- DE-C- 473 487
- DE-C- 832 044
- FR-A- 694 467
- US-A1- 2009 118 656
- US-A1- 2012 253 235

## Beschreibung

Die Erfindung betrifft ein Führungsgelenk für eine Gelenksorthese zur physiologischen Führung eines anatomischen Gelenks, wobei das Führungsgelenk einen ersten Gelenksschenkel und einen zweiten Gelenksschenkel aufweist und die Gelenksschenkel über eine erste Linearführung und eine zweite Linearführung miteinander verbunden sind, wobei jede der Linearführungen eine als Schlitz oder Nut ausgebildete Führungsbahn in dem zweiten Gelenksschenkel aufweist, in die jeweils ein auf dem ersten Gelenksschenkel angeordnetes Gleitelement eingreift, und wobei die beiden Gleitelemente räumlich voneinander getrennt und beabstandet sind und von dem ersten Gelenksschenkel abstehen.

Die Erfindung betrifft weiters eine Gelenksorthese für ein Kniegelenk mit einer Oberschenkelmontage und einer Unterschenkelmontage, die über ein äußeres Führungsgelenk und ein inneres Führungsgelenk miteinander verbunden sind.

Die Beugebewegung des menschlichen Kniegelenks ist ein komplexes kinematisches Zusammenspiel verschiedener Translations- und Rotationsbewegungen, die sich durch das Abrollen und Gleiten der Femurkondylen (mediale und laterale Kondyle) des Oberschenkelknochens (Femur) auf dem gegenüberliegenden Tibiaplateau des Unterschenkelknochens ergeben. Der Zusammenhalt von Tibia und Femur wird durch Bänder, Sehnen und Muskeln gewährleistet, die nicht nur die Beugebewegung, sondern auch eine relative achsiale Verdrehung sowie eine seitliche Winkelverstellung der Tibia gegenüber dem Femur zulassen (in etwa vergleichbar mit einer O-Bein und X-Bein Stellung).

Bei einer vollständigen Flexion (also einer Schwenkbewegung von einem Winkel von 0° - gestrecktes Bein - bis zu einem Winkel von etwa 135° oder mehr - vollständig abgewinkeltes Bein) schwenkt der Oberschenkel nicht nur um eine in Bezug auf den Unterschenkel feststehende Schwenkachse, sondern der Oberschenkel bewegt sich in Bezug auf das Tibiaplateau auch gleichzeitig nach hinten (posterior). Diese Versatzbewegung nach Hinten beträgt bei einem durchschnittlich groß ausgebildeten ausgewachsenen Kniegelenk bis zu etwa 12 - 14 mm. Bei einer vollständigen Extension, also einer Schwenkbewegung von einem Winkel von 135° und mehr bis zur Ausgangsstellung von 0°, dreht sich der Unterschenkel (Tibia) von oben betrachtet um ca. 15° um seine eigene Achse nach außen (lateral). Gleichzeitig kann sich die Tibia auch in einem kleinen Winkelbereich seitlich zum Femur bewegen, in etwa vergleichbar mit einer O-Bein oder X-Bein Stellung.

Während des Abwinkelns vollzieht der Oberschenkel in der Gelenksebene am Anfang, das heißt in den ersten ca. 30° der Beugung, nahezu eine reine Rotation um eine feststehende Rotationsachse, und erst danach setzt zusätzlich die Translationsbewegung posterior und nach unten ein.

Nach Knieoperationen ist es für einen schnellen Heilungsprozess erforderlich, vor Allem das Kniegelenk gezielt physiologisch zu führen. Auch die Beugebewegung sollte vollständig oder nach Bedarf winkelmäßig eingeschränkt möglich sein. Diese Funktion wird von Kniegelenksorthesen ausgeführt, die aus einer Oberschenkelmontage, einer Unterschenkelmontage und einem zwischen Ober- und Unterschenkelmontage angeordneten Gelenksmechanismus aufgebaut sind, wobei die Ober- und Unterschenkelmonategen herkömmlicher Weise mit Bändern oder mit Hartschalen ausgeführt werden.

Vor Allem die physiologische Führung wird von vielen Kniegelenksorthesen des Standes der Technik nur wenig zufriedenstellend gelöst.

Bei herkömmlichen Kniegelenksorthesen besteht der Gelenksmechanismus beispielsweise aus zwei monozentrischen Drehgelenken, die an den gegenüberliegenden Seiten des Kniegelenks angeordnet sind, und eine Drehachse durch das Kniegelenk definieren. Diese Drehbewegung um eine fixe Drehachse widerspricht jedoch der physiologischen Kinematik des anatomischen Knies. Da eine Drehung nicht gleichzeitig um zwei verschiedene Achsen erfolgen kann, entsteht zwischen dem anatomischen Knie und der Orthese eine Differenzbewegung, welche zu Abhebungen und Verschiebungen der Orthese auf dem Bein des Patienten führt und von den Patienten vor Allem beim stärkeren Abwinkeln des Knies als äußerst unangenehm empfunden wird. Diese Differenzbewegung kann auch auf den Heilprozess negative Auswirkungen haben, da die Bänder und Sehnen zusätzlich belastet werden.

Üblicherweise wird in der Anwendung handelsüblichen Knie-Orthesen eine "Kompromissachse" gesucht welche so gewählt wird, dass sich während einer kompletten Beugung zwischen dem monozentrischen Orthesengelenk und dem anatomischen Kniegelenk die geringsten Verschiebungen ergeben. Der "Kompromissbereich" ist eine begrenzte kleine Fläche an der Innen- und Außenseite des Knies welche mit dem variablen Rotationsbereich des anatomischen Knies am besten fluchtet. Dieser "Kompromissbereich" ist gewöhnlich im hinteren Drittel der Femurkondylen zu finden. Wird die Drehachse außerhalb dieser begrenzten Fläche positioniert, entstehen ganz erhebliche Verschiebungen und Abhebungen der Orthese auf dem Bein des Patienten. In der Praxis kann eine nachhaltig fluchtende Montage der Orthese auf dem Bein des Patienten jedoch kaum erreicht werden.

Zahlreiche Versuche, physiologisch korrekte Gelenke für Kniegelenksorthesen zu schaffen, sind daran gescheitert, dass die oben beschriebenen komplexen Bewegungsabläufe des Kniegelenks zwar ausreichend bekannt sind, aber bisher mechanisch nicht ausreichend umgesetzt wurden.

DE 832044 C wird als nächstliegender Stand der Technik angesehen und offenbart ein Schienen-Kniegelenk mit einem mit dem Oberschenkelteil verbundenen Mittelteil, an dem beidseitig abstehend zwei Führungsrollen angeordnet sind. Die Führungsrollen werden in Nuten geführt, die in beidseitig des Mittelteils angeordnete Gelenkbacken des Unterschenkels vorgesehen sind. Die Nuten sind in etwa in einem rechten Winkel zueinander angeordnet und bilden eine im Wesentlichen L-Förmige Konfiguration aus.

FR 694467 A offenbart ein künstliches Kniegelenk mit einem zum Oberschenkel gehörigen Mittelteil und zwei zum Unterschenkel gehörigen Seitenteilen. Der Mittelteil weist eine L-förmige Führungsnut auf, in der zwei Führungsschrauben geführt sind, die in die Seitenteile eingeschraubt sind.

US 2012/0253235 A1 offenbart eine Kniegelenksorthese mit Steuerungskurven, in denen Führungsbolzen geführt sind. Die Kniegelenksorthese verwendet eine Kurvensteuerung, um eine Verschiebung des Rotations-Zentrums während der Beugung zu bewirken. DE3504633 A1 offenbart eine orthotische Kniegelenkanordnung, bei der zwei Führungsstifte eines ersten Gelenksteils in Ausnehmungen eines zweiten Gelenksteils eingreifen. Die Ausnehmungen begrenzen die Auslenkbewegungen des Gelenks, wobei die Relativpositionen zwischen den Gelenksschenkeln in Bezug auf den Beugungswinkel nicht eindeutig definiert sind, sondern ein Spiel ermöglichen.

DE473487 C offenbart ein Kniegelenk für künstliche Beine mit einer zweifachen Kurvengleitführung, bei der zwei Führungsstifte eines Gelenksteils in zwei zugehörige, einander mit ihren rückwärtigen Enden überschneidende Führungsnuten des anderen Gelenksteils derart geführt sind, dass bei der Bewegung des Knies die entsprechend gekrümmte Stützkante am unteren Ende der Oberschenkelschiene auf einer Stützrolle der Unterschenkelschiene entlangzugleiten vermag.

WO2013040354 A1 offenbart eine Kniegelenksorthese, deren Gelenke einen fixen Drehpunkt aufweisen.

Die US 2013/0018293 A1, die vom gegenständlichen Anmelder eingereicht wurde, offenbart mehrere Ausführungsformen von Gelenken für Kniegelenksorthesen, mit denen es erstmals gelungen ist, die kinematischen Vorgaben des Kniegelenks zufriedenstellend nachzubilden. Eine in der US 2013/0018293 A1 offenbarte Ausführungsform eines Gelenks weist zwei flache, aneinanderliegende Gelenksschenkel auf, wobei in einem der Gelenksschenkel eine im Wesentlichen längs zur Schenkelachse verlaufende, breite Führungsnut und am Boden der Führungsnut ein im Wesentlichen quer zur Schenkelachse verlaufende schmaler Führungsschlitz eingebracht sind. Die andere Schenkelachse weist ein kreisrundes Plateau auf, dessen Durchmesser der Breite der Führungsnut entspricht. Auf dem Plateau ist weiters ein Bolzen angeordnet, dessen Durchmesser der Breite des Führungsschlitzes entspricht. Das Plateau wird so in die Führungsnut eingesetzt, dass der Bolzen in dem Führungsschlitz angeordnet ist. Durch die beiden, im Wesentlichen quer zueinander angeordneten Führungen ergibt sich zwischen den beiden Gelenksschenkeln eine kinematische Bewegung, die der Kniebewegung sehr genau entspricht. Die Translationsbewegung des oberen Gelenksschenkels, also der Gelenksschenkel, der die Bewegung des Oberschenkels nachvollziehen soll, entspricht dabei im Wesentlichen der Länge des Führungsschlitzes.

Nachteilig ist jedoch die komplexe Herstellung dieses Gelenks. Aufgrund der beiden seitlich nebeneinander angeordneten Gelenksschenkel sind Begrenzungsstifte, die in Bohrungen eingesetzt werden, um die Beugebewegung winkelmäßig zu begrenzen, in nachteiliger Weise auf Biegung beansprucht.

Es ist das Ziel der gegenständlichen Erfindung ein Führungsgelenk und eine mit diesem Führungsgelenk versehene Kniegelenksorthese bereitzustellen, die den Stand der Technik weiter verbessert. Insbesondere soll ein Führungsgelenk geschaffen werden, das klein, leicht und einfach herzustellen ist. Zwischen dem ersten Gelenksschenkel und dem zweitem Gelenksschenkel soll sich eine entsprechende physiologische Gelenksbewegung ergeben.

Diese und weitere Ziele werden erfindungsgemäß durch ein Führungsgelenk der eingangs genannten Art erreicht, bei dem die Führungsbahnen sich kreuzen, wobei während der ersten 30° der Gelenksbeugung aufgrund der Zwangsführung der Gleitelemente das erste Gleitelement im Bereich des vorderen Endes der ersten Führungsbahn verbleibt, während sich das zweite Gleitelement in der zweiten Führungsbahn abwärts bewegt, und das erste Gleitelement in den Führungsbahnen seine Position in der ersten Führungsbahn nicht wesentlich verändert. Dies erlaubt die Herstellung eines kostengünstigen und stabilen Führungsgelenks, das auch starken statischen und dynamischen Belastungen, wie sie etwa bei Kniegelenksorthesen auftreten, dauerhaft widersteht. Die beiden Drehachsen der Gleitelemente sind dabei parallel, jedoch seitlich zueinander versetzt. Dadurch ist in den gekreuzten Führungsbahnen die Lage der Gleitelemente bei jedem Beugungswinkel eindeutig definiert.

Der Abstand der beiden Drehachsen und die Anordnung und Länge der beiden Führungsbahnen ist so gewählt, dass die sich ergebende Bewegung beim Abwinkeln des Führungsgelenks der physiologischen Beugebewegung eines durchschnittlich groß ausgebildeten Femurkopfes möglichst exakt entspricht.

In einer bevorzugten Ausführungsform können die Gleitelemente an zwei gegenüberliegenden Seiten des ersten Gelenksschenkels angeordnet sein. Dabei können die Gleitelemente in unterschiedliche Richtungen von dem Gelenksschenkel nach Außen abstehen oder sie können von zwei gegenüberliegenden Innenflächen des Gelenksschenkels in entgegengesetzte Richtungen nach Innen hin abstehen.

In einer vorteilhaften Ausführungsform kann der erste oder zweite Gelenksschenkel Bohrungen zum Einsetzen eines Begrenzungsstifts aufweisen. Dadurch lässt sich der mögliche Bereich der Beugungswinkel des Führungsgelenks auf einfache und effektive Weise beschränken. Bei einem Gelenksschenkel, der aus zwei Seitenteilen aufgebaut ist, können die Seitenteile deckungsgleiche Bohrungen zum Einsetzen eines Begrenzungsstifts aufweisen.

Bei einer weiteren vorteilhaften Ausgestaltung der Erfindung können die Gleitflächen auf den Gleitelementen und/oder an den Führungsbahnen mit einer Reibungs- und Abnutzungsverringernden Oberflächenbeschichtung versehen sein. Eine Oberflächenbeschichtung verringert einerseits den Verschleiß des Führungsgelenks und kann eine aufwendige Härtung gegebenenfalls ersetzen.

Das erfindungsgemäße Führungsgelenk kann auch in Prothesen vorteilhaft verwendet werden.

Die eingangs genannte Gelenksorthese für ein Kniegelenk, die an beiden Seiten mit einem erfindungsgemäßen Führungsgelenk versehen ist, ermöglicht eine physiologisch korrekte Führung des Gelenks über den gesamten Beugungsbereich. Aufgrund der erreichbaren geringen Größe der Führungsgelenke kann die Gelenksorthese ein sehr geringes Gewicht aufweisen und gut an die Körperform angepasst werden, sodass zusätzlich ein guter Tragekomfort gewährleistet ist.

In vorteilhafter Weise kann dabei eine äußere Oberschenkelschiene der Oberschenkelmontage über ein erstes Scharnier mit dem äußeres Führungsgelenk verbunden sein, und eine innere Unterschenkelschiene der Unterschenkelmontage kann über ein zweites Scharnier, eine Zwischenschiene und ein drittes Scharnier mit dem inneren Führungsgelenk verbunden sein. Dies erlaubt eine vorteilhafte Anpassung der Gelenksortheses an unterschiedliche Beinformen. Gegebenenfalls können, je nach Bedarf, weniger oder mehr als die oben angeführten Scharniere vorgesehen sein. Die Scharniere können auch ein einfaches, platzsparendes Zusammenlegen der nicht verwendeten Gelenksorthese ermöglichen.

In einer bevorzugten Ausführungsform kann das innere Führungsgelenk einteilig mit der inneren Oberschenkelschiene ausgebildet sein und/oder das äußere Führungsgelenk kann einteilig mit der äußeren Unterschenkelschiene ausgebildet sein.

Die gegenständliche Erfindung wird nachfolgend unter Bezugnahme auf die Figuren 1 bis 5 näher erläutert, die beispielhaft, schematisch und nicht einschränkend vorteilhafte Ausgestaltungen der Erfindung zeigen. Dabei zeigt
Fig.1 eine schematische Darstellung des Bewegungsablaufs eines menschlichen Kniegelenks bei einer Beugebewegung des Knies;
Fig. 2 ein erfindungsgemäßes Führungsgelenk in einer auseinandergezogenen schaubildlichen Darstellung;
Fig. 3 das Führungsgelenk der Fig. 2 in einer weiteren auseinandergezogenen schaubildlichen Darstellung aus einer anderen Perspektive;
Fig. 4a bis 4h eine schematische Darstellung des Bewegungsablaufs des in Fig. 2 und 3 dargestellten Führungsgelenks;
Fig. 5 und 6 eine alternative Ausführungsform des erfindungsgemäßen Führungsgelenks in zwei Rissdarstellungen;
Fig. 7 zwei erfindungsgemäße Führungsgelenke mit den daran angeschlossenen Schienen einer Kniegelenksorthese; und
Fig. 8 eine Darstellung der kinematischen Zusammenhänge anhand der Bewegung der Gleitelemente in ihren Führungsbahnen während einer Beugebewegung.

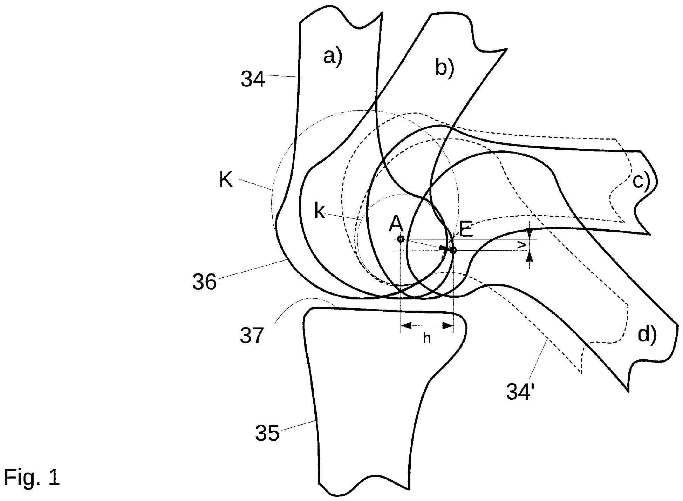

Zur Veranschaulichung des Bewegungsablaufes, der sich bei der Beugung eines menschlichen Kniegelenks ergibt, zeigt Fig.1 diesen Bewegungsablauf anhand von vier unterschiedlichen Beugungswinkeln des Knies in jeweils einer Schnittansicht durch Femur 34 (Oberschenkelknochen) und Tibia 35 (Schienbein), wobei die Schnittebene der Gelenksebene entspricht, die normal zur Gelenksachse verläuft. Die weiteren Elemente des Kniegelenks, wie zum Beispiel die Kniescheibe, der Meniskus, das Wadenbein oder die zahlreichen Bänder sind der Übersichtlichkeit halber in Fig. 1 nicht dargestellt.

Ausgehend von einem gestreckten Bein (Position a zeigt Tibia- und Femurkopf bei einem Beugungswinkel von 0°), zeigen die nachfolgenden Positionen b, c und d jeweils eine bei 30°, 90° und 135° weiter gebeugte Position des Kniegelenks. Ein gesundes Kniegelenk kann noch weiter abgewinkelt werden, wobei die maximal mögliche Beugung im Allgemeinen bei einem Winkel zwischen etwa 140° bis 150° liegt.

Während der Beugebewegung des Knies gleiten die Kondylen 36 des Femur 34 auf dem angrenzenden pfannenartigen Tibiaplateau 37 der Tibia 35. Wesentlich für die Bewegung ist somit die Form der Kondylen 36, deren Verlauf in der Gelenksebene vereinfacht als zwei ineinander übergehende Schmiegekreise K und k veranschaulicht werden kann, wobei der größere Schmiegekreis K einen größeren Radius aufweist, als der Radius des kleineren Schmiegekreises k. Bei der Beugebewegung gleiten die Kondylen 36 anfänglich, also vom gestreckten Bein (0°) bis zu einer Beugung von etwa 30°, nahezu ausschließlich entlang des größeren Schmiegekreises K, sodass sich theoretisch für den Femur 34 eine nahezu reine Drehbewegung um den Mittelpunkt des größeren Schmiegekreises K ergibt.

Tatsächlich befindet sich der gewählte Drehpunkt für alle monozentrischen Gelenke in einem "Kompromissbereich" welcher im hinteren Drittel der Femurkondylen, innerhalb des kleinen Schmiegungskreises k, zu finden ist.

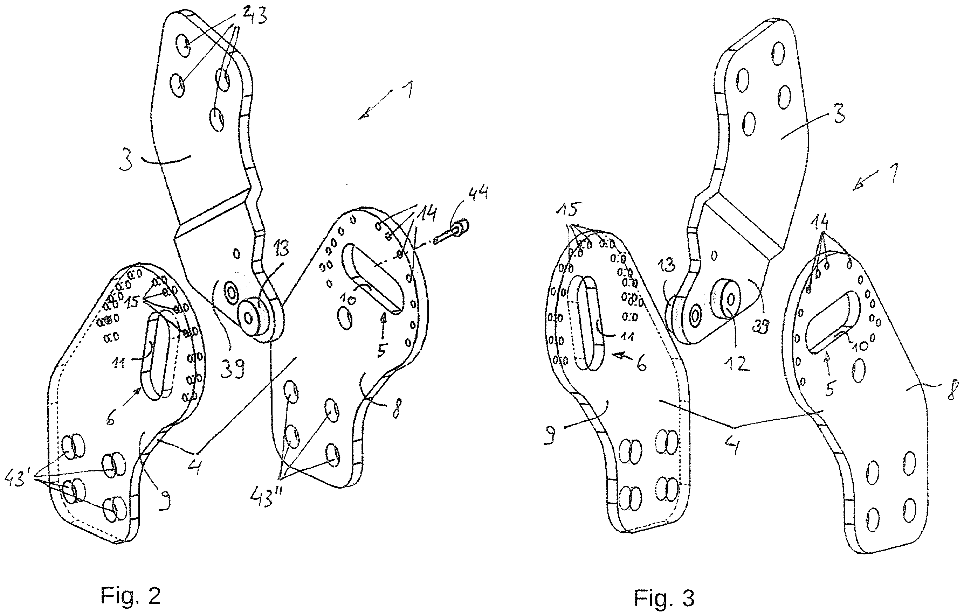

Ab Position b findet ein Übergang vom großen Schmiegekreis K zum kleinen Schmiegekreis k statt wobei die Kondylen auf dem Tibiaplateau zusätzlich zur Gleitbewegung auch zu rollen beginnen und sich das Rotationszentrum progressiv nach posterior verschiebt. (Diese Verschiebung entspricht der weiter unten beschriebenen Verschiebung eines Gleitelements 12 entlang einer Führungsbahn 10 einer Linearführung 5, in Fig. 2 bis 4).

Die anfängliche Positionen A des variablen Drehzentrums gegenüber dem Tibiaplateau verändert sich während der Beugebewegung und wandert bis zu einer Endposition E, die in Fig. 1 für den Beugewinkel von 135° dargestellt ist. Wie aus Fig. 1 zu erkennen ist, verändern sich auch die Berührungspunkte zwischen den Femur Kondylen und dem Tibiaplateau, und wandern während der Beugebewegung deutlich nach hinten (posterior) was ab einem Beugungswinkel von 30° durch eine zusätzliche Rollbewegung zu erklären ist.

Für die Positionen c (90° Beugung) und d (135° Beugung) ist in Strichlinien jeweils die Lage des Femurs 34' dargestellt, die sich (von Position a ausgehend) bei einer reinen Rotation des Femur 34 um einen fixen Drehpunkt ergeben würde, wobei als virtuelles Drehzentrum die anfängliche Position A des variablen Drehzentrums gewählt wurde. Dabei ist zu erkennen, dass sich die tatsächliche Position des Femur 34 in der Position d gegenüber der virtuellen Lage 34' um einen horizontalen Versatz h und einen vertikalen Versatz v "verschoben" hat.

Dieser horizontale und vertikale Versatz ist die Ursache dafür, dass eine Kniegelenksorthese, die ein reines Drehgelenk aufweist, dazu neigt, in der abgewinkelten Position, d.h. ab einem Beugungswinkel von 30°, den Oberschenkel vom Unterschenkel "wegzudrücken", sodass zwischen Femur 34 und Tibia 35 eine Zugbelastung entsteht, die die Bänder, die eigentlich geschont werden sollten, zusätzlich beanspruchen würde. Bei Kniegelenksorthesen mit fixer Drehachse wird daher üblicherweise der maximale Beugewinkel eingeschränkt. Damit ist zwar ein Gehen noch möglich, Bewegungen, die einen größeren Beugewinkel erfordern werden von der Orthese jedoch verhindert. Eine sportliche Betätigung, etwa Laufen, Radfahren, Gymnastik oder Schwimmen, ist mit einem eingeschränkten Maximalwinkel der Beugung kaum möglich. Auch ein gezieltes Training zum Muskelaufbau und zur Wiedererlangung der vollen Gelenksbeweglichkeit nach einer Operation können mit der Orthese nicht befriedigend ausgeführt werden. Übungen, die einen größeren Beugewinkel erfordern können vom Patienten nicht mehr alleine durchgeführt werden. Stattdessen muss die Übung bei abgelegter Orthese mithilfe eines Therapeuten durchgeführt werden, der das Gelenk während der Übung manuell stützt. Mithilfe einer ergonomisch korrekten Orthese könnte der Patient viele Übungen, ohne die kostenintensive professionelle Unterstützung durch einen Therapeuten, häufiger und regelmäßiger durchführen.

Um ein physiologisches Führungsgelenk zu erzeugen, muss daher der in Fig. 1 dargestellte Bewegungsablauf möglichst getreu nachgebildet werden, wobei insbesondere das Erzielen des verhältnismäßig großen horizontalen Versatzes h von Gelenken mit einem monozentrischen Gelenk technisch nicht möglich ist.

Fig. 2 und Fig. 3 zeigen ein erfindungsgemäßes Führungsgelenk 1 aus zwei unterschiedlichen Perspektiven. Das Führungsgelenk 1 besteht aus einem einteiligen oberen Gelenksschenkel 3 und einem zweiteiligen unteren Gelenksschenkel 4. Der obere Gelenksschenkel 3 besteht aus einer im Wesentlichen flachen Platte, die einen Führungsbereich 39 aufweist, von dem auf einer Seite abstehend ein erstes Gleitelement 12 und von der gegenüberliegenden Seite abstehen ein zweites Gleitelement 13 angeordnet sind. Die Gleitelemente 12, 13 sind jeweils als kreisförmige Scheiben ausgebildet, die an der Fläche des Führungsbereichs 39 befestigt sind. Die Scheiben können fix montiert oder als Rollen ausgebildet sein, wobei die Achsen der beiden Gleitelemente 12, 13 versetzt zueinander angeordnet sind. Die beiden Gleitelemente sind voneinander räumlich getrennt und weisen, bezogen auf die Gelenksebene, keine Überschneidungen auf. Die Oberfläche der Gleitelemente 12, 13 weist eine hohe Härte und eine geringe Reibung auf, wobei dies entweder durch eine Härtung der Oberfläche oder durch eine Beschichtung erzielt werden kann.

Der untere Gelenksschenkel 4 besteht aus einem ersten Seitenteil 8 und einem zweiten Seitenteil 9, die im Wesentlichen die gleichen Außenkonturen aufweisen. In dem ersten Seitenteil 8 ist eine schlitzförmige erste Führungsbahn 10 für das erste Gleitelement 12 vorgesehen, und in dem zweiten Seitenteil 9 ist eine schlitzförmige zweite Führungsbahn 11 für das zweite Gleitelement 13 vorgesehen. Die beiden Gleitelemente 12, 13, die in jeweils einer Führungsbahn 10, 11 gleiten, bilden mit diesen an jeder Seite des oberen Gelenksschenkels 3 jeweils eine Linearführung 5, 6 aus. Die Führungsbahnen 10, 11 sind als einfache Schlitze in den Seitenteiles 8, 9 ausgebildet, wobei auch einfache oder profilierte Nuten oder andere, komplexere Führungsbahnen für die Gleitelemente 12, 13 des oberen Gelenksschenkels vorgesehen sein können. Die in den Führungsbahnen 10, 11 vorgesehenen Gleitoberflächen können, wie die entsprechenden Oberflächen der Gleitelemente 12, 13 beschichtet oder gehärtet sein, um die Gleiteigenschaften und die Langlebigkeit des Gelenks zu verbessern.

Der obere Gelenksschenkel 3 und die beiden unteren Seitenteile 8, 9 weisen jeweils Montagebohrungen 43 auf, mit denen das Führungsgelenk an benachbarten Strukturen, beispielsweise an der Oberschenkelmontage bzw. der Unterschenkelmontage einer Kniegelenksorthese, befestigt werden kann. Anstelle der Montagebohrungen 43 können auch beliebige andere Arten der Befestigung vorgesehen sein. Dabei kann zwischen den beiden Seitenteilen 8, 9 ein Distanzelement vorgesehen sein, sodass der Abstand zwischen den Seitenteilen 8, 9 der Dicke des Führungsbereichs 39 des oberen Gelenksschenkels 3 plus einer Spielzugabe entspricht. Vorzugsweise kann für den unteren Gelenksschenkel 4 die benachbarte Struktur, also etwa eine Schiene der Unterschenkelmontage, an der das Führungsgelenk befestigt wird, gleichzeitig die Funktion des Abstandelements ausüben.

In vorteilhafter Weise kann der obere Gelenkschenkel 3 des inneren Führungsgelenkes aus einem Stück mit der Oberschenkelmontage einer Kniegelenksorthese gefertigt werden, sodass sich die Montagebohrungen erübrigen. Dasselbe trifft auch auf das äußere Führungsgelenk zu, wobei ein Seitenteil des unteren Gelenkschenkel 4 gemeinsam mit der Unterschenkelmontage aus einem Stück gefertigt werden kann. In diesem Fall muss allerdings das zweite Seitenteil mit Montagebohrungen versehen sein um das Führungsgelenk zusammenzuhalten.

Weiters sind in die Seitenteile 8, 9 im Randbereich eine Anzahl an Bohrungen 14, 15 eingebracht, wobei jeder Bohrung 14 des ersten Seitenteils 8 eine achsgleiche Bohrung 15 in dem zweiten Seitenteil 9 zugeordnet ist, sodass sich mehrere Bohrungspaare ergeben. Durch jedes Bohrungspaar 14, 15 kann ein Begrenzungsstift 44 geschoben werden (dargestellt in Fig. 2), der die Bewegung des oberen Gelenksschenkels 3 bei einem bestimmten Winkel beschränkt. Der Begrenzungsstift 44 kann beispielsweise mit einem Gewinde versehen sein, das in ein in der Bohrung 15 vorgesehenes Gewinde eingeschraubt wird, um den Begrenzungsstift 44 zu fixieren.

Die Bezeichnungen "oben" und "unten" beziehen sich lediglich auf die in Fig. 2 und 3 dargestellte Ausrichtung, es ist jedoch dem Fachmann klar, dass das Gelenk auch umgekehrt verwendet werden kann, sodass der "untere" Gelenksschenkel 4 der Fig. 2 und 3 ebensosehr als oberer Gelenksschenkel in eine Gelenksorthese eingebaut werden kann. In dem Fall müsste auch der Verlauf der Linearführungen 5, 6 entsprechend angepasst werden. Die Begriffe "oben" und "unten" dienen lediglich der Vereinfachung der Beschreibung und sind nicht einschränkend auszulegen. Gleiches gilt für die Begriffe "innen" (also die dem Kniegelenk zugewandte Seite) und "außen" (also die dem Kniegelenk abgewandte Seite).

Die beiden Führungsbahnen 10, 11 sind, bezogen auf die Gelenksebene, überkreuzend angeordnet, wobei sich durch die kreuzweise Anordnung und die beiden versetzt angeordneten Gleitelemente 12, 13 für jeden Beugungswinkel eine eindeutig festgelegte Relativposition zwischen oberem Gelenksschenkel 3 und unterem Gelenksschenkel 4 ergibt. Die erste Linearführung 5 ist im Wesentlichen horizontal angeordnet, wobei das hintere, d. h. der Beugungsinnenseite (also z.B. der Kniekehle) zugewandte Ende der Linearführung ein wenig tiefer angeordnet ist, als das vordere (also zum Beispiel der Kniescheibe) zugewandte Ende. Dies ist darauf zurückzuführen, dass der Mittelpunkt des keinen Schmiegungskreises k der Femurkondylen in der Ausgangsstellung bei 0° Beugung vom Tibiaplateau weiter entfernt ist, als in der Endstellung bei 135°. Die zweite Linearführung 6 ist normal auf die erste Linearführung 5, d.h. im Wesentlichen vertikal ausgerichtet. Der Winkeln zwischen erster und zweiter Linearführung 5, 6 beträgt etwa 90°, die Linearführungen können jedoch auch in einem spitzen oder stumpfen Winkel zueinander angeordnet sein.

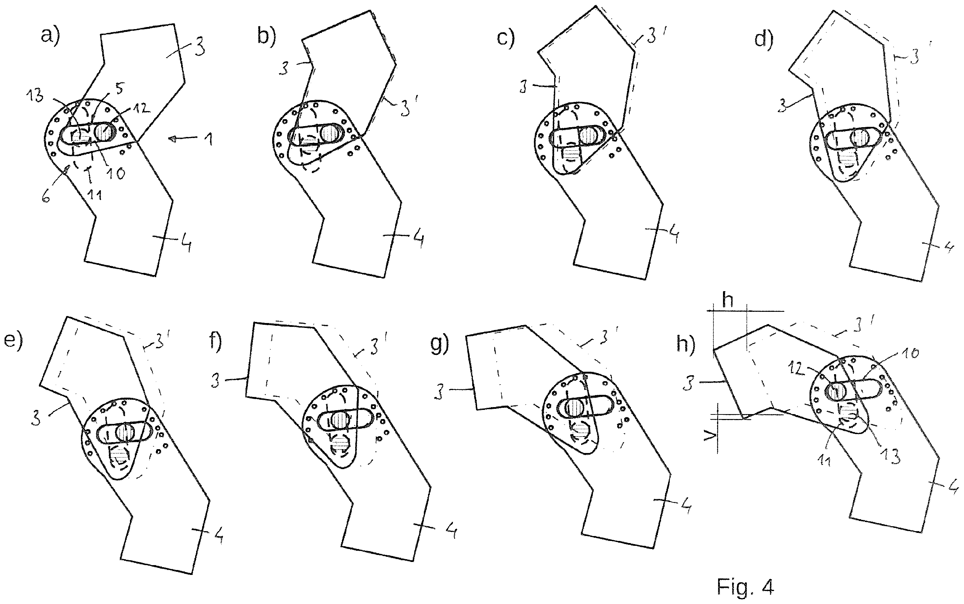

Die Relativbewegung beim Abwinkeln des Führungsgelenks 1 ist in Fig. 4a bis 4h dargestellt, wobei die wesentlichen Elemente des Führungsgelenks 1 in die Gelenksebene projiziert dargestellt sind. Dabei ist insbesondere die zwangsgeführte Bewegung der mit dem oberen Gelenksschenkel 3 fix verbundenen Gleitelemente 12, 13 in den kreuzweise angeordneten Führungsbahnen 10, 11 zu beachten. In den Positionen der Fig. 4a bis 4c, die eine Beugung von etwa 20°, 35° bzw. 40° entsprechen, verbleibt das erste Gleitelement 12 im Bereich des vorderen Endes der ersten Führungsbahn 10, während sich das zweite Gleitelement 13 in der zweiten Führungsbahn 11 abwärts bewegt. Die Bewegung des oberen Gelenksschenkels 3 (bezogen auf den feststehenden unteren Gelenksschenkel 4) entspricht dabei im Wesentlichen einer Schwenkbewegung um das erste Gleitelement 12, das in diesem Bereich der Beugebewegung als im Wesentlichen feststehende Gelenksaxe angesehen werden kann. Diese Beugebewegung entspricht im Wesentlichen dem physiologischen Bewegungsabschnitt beim anatomischen Kniegelenk, bei dem die Kondylen 36 entlang dem ersten Schmiegekreis K gleiten (vergleiche Fig. 1).

Beginnend mit einem Beugungswinkel von 30° der in Fig. 4c dargestellten Position, und verstärkt in den Fig. 4d bis 4e, beginnt bei einer weiteren Beugung des Führungsgelenks 1 das erste Gleitelement 12 sich in der ersten Führungsbahn 10 nach hinten (posterior, d.h. weg von dem vorderen Ende der Führungsbahn 10) zu bewegen, Bei einem Beugungswinkel von 90° hat das zweite Gleitelement 13 in der Führungsbahn 11 den tiefsten Punkt erreicht (unteres Ende der Führungsbahn 11) und bewegt sich dann bei weiterer Flexion wieder so lange nach oben, bis das erste Gleitelement 12 bei voller Flexion am hinteren Ende der ersten Führungsbahn 10 ansteht. Bei der anschliesender Extension bewegt sich das zweite Gleitelement 13 wieder nach unten wobei es bei einem Beugungswinkel von 90° wieder den tieften Punkt erreicht und sich bei weiterer Extension wieder ganz nach oben in die Ausgangsstellung bei 0° bewegt. (das Gleitelement 13 oszilliert bei einer vollen Flexion und Extension zwischen 90° und 135°).

Um den Drehpunkt während der ersten 30° in einer fixen Position zu halten, wurde das zweite Gleitelement 13 in einem Winkel von 15° oberhalb des Gleitelementes 12 in der zweiten Führungsbahn 11 platziert. Würde sich nun das Gleitelement 13, welches sich gemeinsam mit dem Gleitelement 12 auf dem dem Oberschenkel zugeordneten Gelenkschenkel 3 befindet, in einem Kreisbogen von 15° um das Gleitelement 12 drehen, dann befinden sich beide Gleitelemente auf der Achse der Führungsbahn 10. Da jedoch die Führungsbahn 11 über die ersten 15° keinen Kreis sondern eine Gerade Linie darstellt und der Abstand der Mittelpunkte der beiden Gleitelemente größer ist als der Abstand vom ersten Gleitelement 12 zur Schnittstelle der beiden Führungsbahnen, wird das erste Gleitelement 12 geringfügig nach außen gedrückt. Um dieses Differenzmaß muss die Führungsbahn 10 verlängert werden um ein blockieren der Bewegung zu verhindern. Bei einer Drehung um weitere 15° wandert das erste Gleitelement 12 wieder um dieses Differenzmaß zurück in die Ausgangsstellung. Nun hat der Gelenkschenkel 3 eine Rotation von 30° ausgeführt ohne dass das erste Gleitelement 12, welches das bewegliche Rotationszentrum darstellt, seine Position wesentlich verändert hat.

Die Linearführung 6 (Führungsbahn 11) könnte jedoch auch gekrümmt verlaufen, sodass sich am Anfang der Beugebewegung (bei einer Beugung zwischen 0° und etwa 30°) eine reine Kreisbewegung um das erste Gleitelement 12 ergeben könnte. Die Linearführung 6 müsste dazu in einem Kreisbogen von 30° um das vordere Ende der ersten Linearführung 5 herum geführt werden und anschließend wieder gerade verlaufen. Die dargestellte Ausführungsform mit gerade verlaufenden Gleitelementen 12, 13 ist jedoch ausreichend, um eine physiologisch korrekte Beugebewegung hinreichend gut nachzuahmen (die Differenzialbewegung des Gleitelementes 12 während der ersten 30° ist unwesentlich und kann vernachlässigt werden).

Um die Beugebewegung, die der obere Gelenksschenkel 3 bezogen auf den feststehenden unteren Gelenksschenkel 4 vollzieht, im Vergleich zu einer reinen Schwenkbewegung um eine feststehende Achse zu veranschaulichen, ist in Fig. 4b bis 4h jeweils mit strichpunktierter Linie eine Lage eines vergleichenden oberen Gelenksschenkels 3' eingezeichnet, die sich ergeben würde, wenn dieser vergleichende obere Gelenksschenkel 3' sich ausgehend von der in Fig. 4a dargestellten Position um eine feststehende Schwenkachse drehen würde. Als feststehende Schwenkachse des vergleichenden oberen Gelenksschenkels 3' wurde die in Fig. 1a dargestellte Position des ersten Gleitelements 12 gewählt. Insbesondere in Fig. 4h ist zu erkennen, dass das erfindungsgemäße Führungsgelenk 1 bei der Beugebewegung sowohl einen horizontalen Versatz h, als auch einen vertikalen Versatz v aufweist, die im Wesentlichen dem horizontalen Versatz h und dem vertikalen Versatz v des anatomischen Kniegelenks entsprechen, wie er in Fig. 1 dargestellt ist.

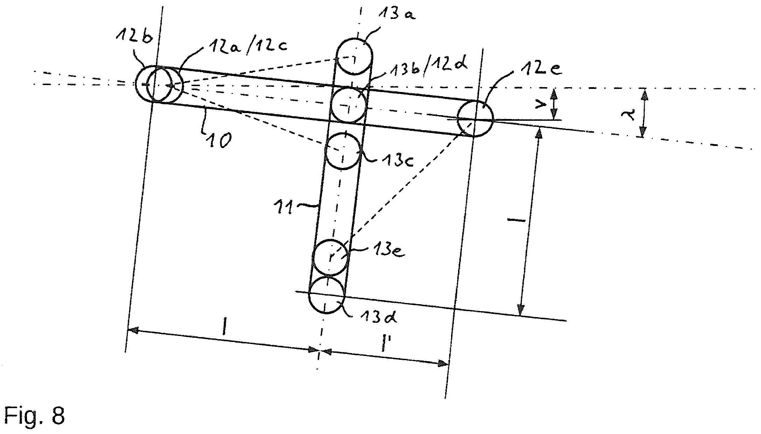

Fig. 8 zeigt die Zwangsführung der Gleitelemente 12, 13 in den Führungsbahnen 10, 11 während einer vollständigen Beugebewegung (0° bis 135°), wobei die Bezugszeichen der Gleitelemente 12, 13 in den einzelnen dargestellten Positionen jeweils gemäß ihrer Abfolge durch die Kleinbuchstaben a, b, c, d und e ergänzt sind.

Aus Fig. 8 ist zu erkennen, wie die erfindungsgemäße Anordnung der Führungsbahnen eine Zwangsführung bewirkt, bei der die Position des ersten Gleitelements während der ersten 30° im Wesentlichen unverändert bleibt. Bei dem dadurch erzielten Bewegungsablauf vollzieht das Gelenk während der ersten 30° der Beugung eine im Wesentlichen reine Drehbewegung um das erste Gleitelement 12, danach bewegt sich das Gleitelement progressiv nach hinten.

Durch die unter 90° gekreuzten Führungsbahnen kann sichergestellt werden, dass das erste Gleitelement 12 nach etwa 30° Beugung wieder an dieselbe Stelle 12c zurückkehrt, die auch den Ausgangspunkt 12a der Beugung (0°) definiert. Dabei vollzieht das Gleitelement lediglich eine geringfügige Auslenkbewegung (bis zur Position 12b), die in Bezug auf den gesamten Bewegungsablauf vernachlässigbar ist.

Das zweite Gleitelement 13 bewegt sich während der ersten 30° der Beugung von der Ausgangslage 13a über die Position 13b zur Position 13c, was mehr als einem Drittel der gesamten Bahnlänge der ersten Führungsbahn 11 entspricht. Das erste Gleitelement 12 hingegen verändert dabei seine Position nur unwesentlich, wobei es nur eine minimale Auslenkung von der Ausgangslage 12a (0°) zu 12b (15°) und wieder zurück zu 12c (30°) vollzieht, die praktisch im Millimeterbereich liegt und nur einem Bruchteil der Länge der zweiten Führungsbahn 10 entspricht. Diese Auslenkung ergibt sich aufgrund des geraden Verlaufs der ersten Führungsbahn und kann aufgrund des geringen Ausmaßes praktisch vernachlässigt werden. Somit ergibt sich für das erste Gleitelement 12 während der ersten 30° der Beugebewegung in der Führungsbahn ein Richtungswechsel, während sich das zweite Gleitelement 13 während dieser ersten 30° der Beugung in der Führungsbahn nur in eine Richtung bewegt.

Erst bei der weiteren Bewegung des zweiten Gleitelements 13 nach unten hin (zu den Positionen 13d und 13e) vollzieht das erste Gleitelement 12 eine progressiv gesteigert Bewegung nach hinten zu den Positionen 12d (im Kreuzungspunkt der beiden Führungsbahnen 10, 11) und 12e (am hinteren Ende der Führungsbahn 10). Dieses Verharren des ersten Gleitelements 12a, 12b, 12c in der Ausgangsposition während der ersten 30° der Beugebewegung (bzw. das Zurückkehren 12c des ersten Gleitelements in die Ausgangsposition 12a nach den ersten 30° der Beugebewegung) und die danach progressiv gesteigerte Bewegung dieses Gleitelements nach hinten (12d, 12e) bis zur maximalen Beugung (ca. 135°) gewährleistet eine Beugebewegung, die der Beugebewegung eines anatomischen Kniegelenks optimal nachempfunden ist.

Wie dies in Fig. 8 dargestellt ist, ist die erste Führungsbahn 10 in Bezug auf die Horizontale unter einem Winkel λ schräg angeordnet, sodass das hintere Ende der Führungsbahn gegenüber dem vorderen Ende um einen vertikalen Versatz v tiefer angeordnet ist. Der Winkel λ beträgt vorzugsweise nur etwa 3°-5°, dies ist jedoch ausreichend um den eingangs genannten Versatz des Femur des anatomischen Kniegelenks gut nachzuahmen.

Das Längenverhältnis der vorderen Länge I der ersten Führungsbahn 10 (also der Länge I, die sich vor dem Kreuzungspunkt der Führungsbahnen befindet) zur hinteren Länge I' dieser Führungsbahn 10 weist im dargestellten Fall ein Verhältnis von etwa 1:0,707 auf. Dies ergibt sich bei der Anordnung der Führungsbahnen unter einem Winkel von 90 Grad aus den Nebenbedingungen, dass der maximale Beugewinkel 135° beträgt und, dass das erste Gleitelement nach einer Beugebewegung von 30° in seine Ausgangslage (12a bzw. 12c) zurücckehrt. Die Länge I entspricht dabei der Entfernung zwischen erstem und zweiten Gleitelement.

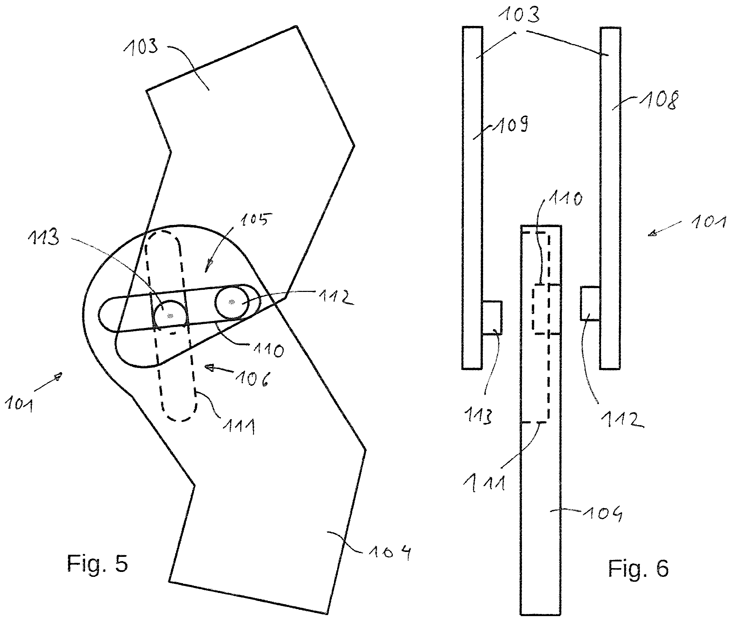

Fig. 5 und 6 zeigen schematisch eine weitere Ausführungsform eines erfindungsgemäßen Führungsgelenks 101, bei dem die Linearführungen 105, 106 gegenüber der in Fig. 2 bis 4 dargestellten Ausführungsform "umgekehrt" wurden, wobei die Gleitelemente 112, 113 an zwei gegenüberliegenden Seitenteilen 108, 109 des oberen Gelenksschenkels 103 nach innen hin abstehend angeordnet sind. Die beiden sich überkreuzenden Führungsbahnen 110, 111 der Linearführungen 105, 106 sind als Nuten in den zwischen den Seitenteilen 108, 109 liegenden unteren Gelenksschenkel 104 eingebracht. Um die Dicke des unteren Gelenksschenkels 4 verringern zu können, überschneiden sich die Nuten der Führungsbahnen 110, 111 in ihrem Kreuzungsbereich.

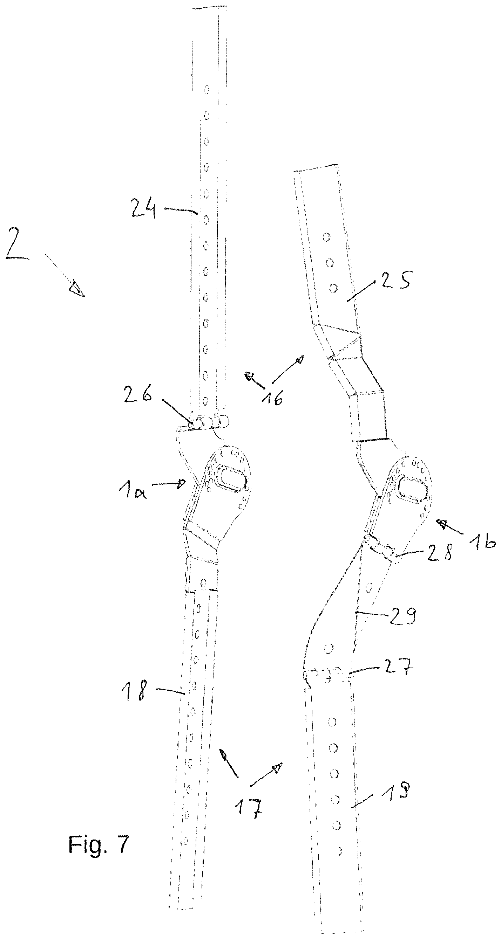

Die Beugebewegung der in Fig. 2 bis 6 dargestellten Führungsgelenke 1, 101 erlaubt es, anatomisch korrekte Kniegelenksorthesen 2 herzustellen, die es dem Therapeuten erlauben, die Vorgaben für einen maximalen (und gegebenenfalls minimalen) Beugewinkel auf Basis der medizinischen Indikation vorzugeben, und nicht aufgrund der Unzulänglichkeit der verwendeten Kniegelenksorthese 2. Die Verwendung eines solchen Führungsgelenks in einer Kniegelenksorthese 2 ist in Fig. 7 veranschaulicht.

Fig. 7 zeigt die Elemente der Oberschenkelmontage 16 und der Unterschenkelmontagen 17 der Kniegelenksorthese 2, die mit den zu beiden Seiten des Kniegelenks anzuordnenden Führungsgelenken 1a und 1b in Verbindung stehen. Das äußere Führungsgelenk 1a verbindet eine äußere Oberschenkelschiene 24 mit einer äußeren Unterschenkelschiene 18 wobei vorteilhafterweise die Verbindung mit der Oberschenkelmontage mittels eines Scharnieres erfolgt. Das innere Führungsgelenk 1b verbindet eine innere Oberschenkelschiene 25 mit einer inneren Unterschenkelschiene 19 wobei die Verbindung mit der Unterschenkelschiene über zwei Scharniere erfolgt, welche über eine Zwischenschiene miteinander verbunden sind. Die Oberschenkelmontage 16 und die Unterschenkelmontage 17 können zur Befestigung am Ober- bzw. Unterschenkel jeweils mit Befestigungselementen, wie etwa Bändern, versehen sein, oder an bekannten Hartschalen befestigt sein (diese sind in Fig. 7 nicht dargestellt).

Die Erfindung wird durch die nachfolgenden Ansprüche definiert.

### Bezugszeichen:

Führungsgelenk 1
Kniegelenksorthese 2
oberer Gelenksschenkel 3
unterer Gelenksschenkel 4
erste Linearführung 5
zweite Linearführung 6
anatomisches Gelenk 7
Seitenteile 8, 9
Führungsbahnen 10, 11
Gleitelemente 12, 13
Bohrungen 14,15
Oberschenkelmontage 16
Unterschenkelmontage 17
äußere Unterschenkelschiene 18
innere Unterschenkelschiene 19
äußere Oberschenkelschiene 24
innere Oberschenkelschiene 25
erstes, zweites, drittes Scharnier 26, 27, 28
Zwischenschiene 29
Femur (Oberschenkelknochen) 34
Tibia (Unterschenkelknochen) 35
Kondylus 36
Tibiaplateau 37
Markierungspunkt 38
Führungsbereich 39
Montagebohrung 43, 43`, 43"
Begrenzungsstift 44

## Patentansprüche

1. Führungsgelenk (1) für eine Gelenksorthese (2) zur Stützung und Führung eines anatomischen Gelenks (7), wobei das Führungsgelenk (1) einen ersten Gelenksschenkel (3) und einen zweiten Gelenksschenkel (4) aufweist und die Gelenksschenkel (3, 4) über eine erste Linearführung (5) und eine zweite Linearführung (6) miteinander verbunden sind, wobei jede der Linearführungen (5, 6) eine als Schlitz oder Nut ausgebildete Führungsbahn (10, 11) in dem zweiten Gelenksschenkel (4) aufweist, in die jeweils ein auf dem ersten Gelenksschenkel (3) angeordnetes Gleitelement (12, 13) eingreift, und wobei die beiden Gleitelemente (12, 13) räumlich voneinander getrennt und beabstandet sind und von dem ersten Gelenksschenkel (3) abstehen, **dadurch gekennzeichnet, dass** die Führungsbahnen (10, 11) sich kreuzen, wobei, in Gebrauch, während der ersten 30° der Gelenksbeugung aufgrund der Zwangsführung der Gleitelemente (12, 13) das erste Gleitelement (12) im Bereich des vorderen Endes der ersten Führungsbahn (10) verbleibt, während sich das zweite Gleitelement (13) in der zweiten Führungsbahn 11 abwärts bewegt, und das erste Gleitelement (12) seine Position in der ersten Führungsbahn (10) nicht wesentlich verändert.

2. Führungsgelenk (1) nach Anspruch 1, **dadurch gekennzeichnet, dass** die beiden Führungsbahnen (10,11), bezogen auf die Gelenksebene, unter einem Winkel von 90° überkreuzend angeordnet sind.

3. Führungsgelenk (1) nach Anspruch 1 oder 2, **dadurch gekennzeichnet, dass** der erste oder der zweite Gelenksschenkel (4) zwei miteinander verbundene Seitenteile (8, 9, 108, 109) aufweist.

4. Führungsgelenk (1) nach Anspruch 3, **dadurch gekennzeichnet, dass** die Gleitelemente (12, 13, 112, 113) an zwei gegenüberliegenden Seiten des ersten Gelenksschenkels (3, 103) angeordnet sind.

5. Führungsgelenk (1) nach Anspruch 3 oder 4, **dadurch gekennzeichnet, dass** der erste oder zweite Gelenksschenkel (3, 4) Bohrungen (14, 15) zum Einsetzen eines Begrenzungsstifts (44) aufweist.

6. Führungsgelenk (1) nach einem der Ansprüche 1 bis 5, **dadurch gekennzeichnet, dass** Gleitflächen auf den Gleitelementen (12, 13) und/oder an den Führungsbahnen (10, 11) mit einer Reibungs- und Abnutzungsverringernden Oberflächenbeschichtung versehen sind.

7. Gelenksorthese (2) für ein Kniegelenk mit einer Oberschenkelmontage (16) und einer Unterschenkelmontage (17), **dadurch gekennzeichnet, dass** die Oberschenkelmontage (16) über ein äußeres Führungsgelenk (1a) und ein inneres Führungsgelenk (1b), jeweils gemäß einem der Ansprüche 1 bis 6, mit der Unterschenkelmontage (17) verbunden ist.

8. Gelenksorthese (2) nach Anspruch 7, **dadurch gekennzeichnet, dass** eine äußere Oberschenkelschiene (24) der Oberschenkelmontage (16) über ein erstes Scharnier (26) mit dem äußeres Führungsgelenk (1a) verbunden ist, und dass eine innere Unterschenkelschiene (19) der Unterschenkelmontage (17) über ein zweites Scharnier (27), eine Zwischenschiene (29) und ein drittes Scharnier (28) mit dem inneren Führungsgelenk (1b) verbunden ist.

9. Gelenksorthese (2) nach Anspruch 7 oder 8, **dadurch gekennzeichnet, dass** das innere Führungsgelenk (1b) einteilig mit der inneren Oberschenkelschiene (24) ausgebildet ist und/oder das äußere Führungsgelenk (1a) einteilig mit der äußeren Unterschenkelschiene (18) ausgebildet ist.

## Claims

1. A guide joint (1) for a joint orthosis (2) for supporting and guiding an anatomical joint (7), wherein the guide joint (1) has a first joint member (3) and a second joint member (4), and the joint members (3, 4) are connected to one another by a first linear guide (5) and a second linear guide (6), wherein each of the linear guides (5,6) has a guide track (10, 11) formed in the second joint member (4) as a slot or groove, in which in each case a sliding element (12, 13) arranged in the first joint member (3) engages, wherein the two sliding elements (12, 13) are spatially separate and spaced apart from one another and protrude from the first joint member (3), **characterized in that** the guide tracks (10, 11) intersect, wherein, in use, during the first 30° of the joint flexion, due to the forced guidance of the sliding elements (12, 13), the first sliding element (12) remains in the region of the anterior end of the first guide track (10), while the second sliding element (13) moves downwards in the second guide track (11) and the first sliding element (12) does not substantially change its position in the first guide track (10) .

2. The guide joint (1) according to claim 1, **characterized in that** the two guide tracks (10, 11) are arranged to intersect one another at an angle of 90° relative to the joint plane.

3. The guide joint (1) according to claim 1 or 2, **characterized in that** the first or second joint member (4) has two side parts (8, 9, 108, 109) that are connected to one another.

4. The guide joint (1) according to claim 3, **characterized in that** the sliding elements (12, 13, 112, 113) are arranged on two opposing sides of the first joint member (3, 103).

5. The guide joint (1) according to claim 3 or 4, **characterized in that** the first and second joint members (3, 4) have boreholes (14, 15) for insertion of a limiting pin (44).

6. The guide joint (1) according to one of claims 1 to 5, **characterized in that** that sliding surfaces on the sliding elements (12, 13) and/or on the guide tracks (10, 11) are provided with a friction- and wear-reducing surface coating.

7. A joint orthosis (2) for a knee joint with an upper leg fastening (16) and a lower leg fastening (17), **characterized in that** the upper leg fastening (16) has an outer guide joint (1a) and an inner guide joint (1b), each of which is connected to the lower leg fastening (17) in accordance with one of claims 1 to 6.

8. The joint orthosis (2) according to claim 7, **characterized in that** an outer upper leg rail (24) of the upper leg fastening (16) is connected to the outer guide joint (1a) by a first hinge (26) and that an inner lower leg rail (19) of the lower leg fastening (17) is connected to the inner guide joint (1b) by means of a second hinge (27), an intermediate rail (29), and a third hinge (28).

9. The joint orthosis (2) according to claim 7 or 8, **characterized in that** the inner guide joint (1b) is formed in a single piece with the inner upper leg rail (24) and/or the outer guide joint (1a) is formed in a single piece with the outer lower leg rail (18).

## Revendications

1. Articulation de guidage (1) pour une orthèse articulaire (2), destinée à soutenir et à guider une articulation (7) anatomique, dans laquelle l'articulation de guidage (1) présente un premier bras d'articulation (3) et un second bras d'articulation (4), et les bras d'articulation (3, 4) sont reliés entre eux par l'intermédiaire d'un premier guide linéaire (5) et d'un second guide linéaire (6), dans laquelle chacun des guides linéaires (5, 6) présente, dans le second bras d'articulation (4), une voie de guidage (10, 11) réalisée sous forme de fente ou de rainure, dans laquelle voie de guidage respectivement un élément coulissant (12, 13) disposé sur le premier bras d'articulation (3) vient en prise, et dans laquelle les deux éléments coulissants (12, 13) sont séparés et espacés spatialement l'un de l'autre et dépassent du premier bras d'articulation (3), **caractérisée en ce que** les voies de guidage (10, 11) se croisent, dans laquelle, lors de l'utilisation, le premier élément coulissant (12) reste dans la région de l'extrémité avant de la première voie de guidage (10) pendant les 30 premiers degrés de flexion de l'articulation en raison du guidage forcé des éléments coulissants (12, 13), tandis que le second élément coulissant (13) se déplace vers le bas dans la seconde voie de guidage 11, et le premier élément coulissant (12) ne change sensiblement pas de position dans la première voie de guidage (10).

2. Articulation de guidage (1) selon la revendication 1,
**caractérisée en ce que** les deux voies de guidage (10,11), par rapport au plan d'articulation, sont disposées de façon à se croiser selon un angle de 90°.

3. Articulation de guidage (1) selon la revendication 1 ou 2,
**caractérisée en ce que** le premier ou le second bras d'articulation (4) présente deux parties latérales (8, 9, 108, 109) reliées entre elles.

4. Articulation de guidage (1) selon la revendication 3,
**caractérisée en ce que** les éléments coulissants (12, 13, 112, 113) sont disposés sur deux côtés opposés du premier bras d'articulation (3, 103).

5. Articulation de guidage (1) selon la revendication 3 ou 4,
**caractérisée en ce que** le premier ou le second bras d'articulation (3, 4) présente des alésages (14, 15) pour l'insertion d'une tige de délimitation (44).

6. Articulation de guidage (1) selon l'une des revendications 1 à 5,
**caractérisée en ce que** des surfaces de coulissement sur les éléments coulissants (12, 13) et/ou sur les voies de guidage (10, 11) sont pourvues d'un revêtement de surface réduisant les frottements et l'usure.

7. Orthèse articulaire (2) pour une articulation du genou, comportant un support de cuisse (16) et un support de jambe (17), **caractérisée en ce que** le support de cuisse (16) est relié au support de jambe (17) par l'intermédiaire d'une articulation de guidage extérieure (1a) et d'une articulation de guidage intérieure (1b), respectivement selon l'une des revendications 1 à 6.

8. Orthèse articulaire (2) selon la revendication 7,
**caractérisée en ce qu'**une attelle extérieure pour cuisse (24) du support de cuisse (16) est reliée à l'articulation de guidage extérieure (1a) par l'intermédiaire d'une première charnière (26), **et en ce qu'**une attelle intérieure pour jambe (19) du support de jambe (17) est reliée à l'articulation de guidage intérieure (1b) par l'intermédiaire d'une deuxième charnière (27), d'une attelle intermédiaire (29) et d'une troisième charnière (28).

9. Orthèse articulaire (2) selon la revendication 7 ou 8,
**caractérisée en ce que** l'articulation de guidage intérieure (1b) est réalisée d'un seul tenant avec l'attelle intérieure pour cuisse (24) et/ou l'articulation de guidage extérieure (1a) est réalisée d'un seul tenant avec l'attelle extérieure pour jambe (18).
